# EUROPEAN PATENT SPECIFICATION

(11) **EP 1 071 402 B1**
(45) Date of publication and mention of the grant of the patent: **13.09.2006**
(21) Application number: 99919195.0
(22) Date of filing: 06.04.1999
(51) Int. Cl.: A61K 9/14, A61K 31/015, C08J 3/12, B01J 2/00, B01J 2/04, B01J 13/00, B01F 3/00, B29B 9/10

(54) **PROCESS FOR THE MANUFACTURE OF (SUB)MICRON SIZED PARTICLES BY DISSOLVING IN COMPRESSED GAS AND SURFACTANTS**
VERFAHREN ZUR HERSTELLUNG VON WENIGER ALS MIKROMETERGROSSEN TEILCHEN DURCH AUFLÖSEN IN DRUCKGAS UND OBERFLÄCHENAKTIVEN STOFFEN
PROCEDE DE PRODUCTION DE PARTICULES SUBMICRONIQUES PAR DISSOLUTION DANS DU GAZ COMPRIME ET DES TENSIOACTIFS

(30) Priority: 09.04.1998 DE 98106534
(43) Date of publication of application: 31.01.2001
(73) Proprietor: F. HOFFMANN-LA ROCHE AG, 4070 Basel (CH)
(72) Inventor: BAUSCH, Alexander, D-79540 Lörrach (DE); HIDBER, Pirmin, CH-4053 Basle (CH)
(74) Representative: Wächter, Dieter Ernst
(86) International application number: PCT/EP1999/002316
(87) International publication number: WO 1999/052504

(56) References cited:
- WO-A-95/21688
- WO-A-97/14407
- WO-A-98/16204
- US-A- 3 998 753
- US-A- 4 734 227
- DONSI, G. ET AL.: "Micronization by Means of Supercritical Fluids: Possibility of Application to Pharmaceutical Field" PHARM. ACTA HELV., vol. 66, no. 5-6, 1991, pages 170-172, XP002900588

## Description

The invention provides a novel process for producing (sub)micron-sized particles of a biologically active compound (pharmaceutical).

In the last years a number of different processes to produce very small particles of a pharmaceutical have been described. (e.g. RESS,GAS,PGSS, SAS). These processes are e.g. described in Journal of Pharmaceutical Sciences Vol. 86, No. 8, August 1997, pp. 885-890 under the title "Pharmaceutical Processing with Supercritical Carbon Dioxide. Thereby the drug is dissolved in a compressed gas and subsequently rapidly expanded mostly into atmospheric pressure. Due to the expansion conditions and to a high surface energy in the gas very small particle sizes (smaller 1 µm) are hard to achieve and to handle. Such high surface areas can only be handled by using a surface modifier to decrease the surface energy. This fact is well known for a long time and used for stabilization of small particles in suspension. (H. Sucker, P. Fuchs, P. Speiser, "Pharmazeutische Technologie", 2. Edition, 1991, Georg Thieme Verlag, Stuttgart/New York, pp 419-424; and Hans Steffen, BT Gattefossé No. 81, 1988, pp. 45-53, "Controlled Precipitation- a Method to Produce Small Drug Particles and to Increase Bioavaibility".)

The International application WO 97/14407 describes a supercritical fluid/compressed fluid based process to produce submicron-sized particles of biologically active compounds which process comprises the steps of:
(1) dissolving a water insoluble biologically active compound in a solvent thereof;
(2) spraying the solution of step (1) into a compressed gas, liquid or supercritical fluid in the presence of a surface modifier dispersed in an aqueous phase.

In another embodiment the process described in WO 97/14407 is carried out comprising the steps of:
(1) dissolving a water insoluble biologically active compound in a compressed fluid;
(2) spraying the compressed fluid of step (1) into an aqueous phase containing a surface modifier.

The process described in WO 97/14407 may be difficult to realize on an industrial scale for the following reasons:
- On an industrial scale it is difficult to reach a uniform distribution of temperature in the connection tubes. Due to such variations in temperature aggregation or flocculation of particles dissolved in the supercritical solution might occur causing clogging of the tubes or spraying-nozzles.
- The solubility of most of the pharmaceutical compounds in liquid or supercritical CO₂ is very low even under high pressure. Therefore the use of additional cosolvents is proposed. Most of these cosolvents are liquids under atmospheric pressure. By spraying the solution containing the pharmaceutical into the liquid phase (e.g. aqueous phase) the fraction of the cosolvent in the liquid phase increases. Therefore the solubility of the compound in the liquid phase also increases. This can destabilize the suspension on an industrial scale.
- In addition, the recycling of the pressurized gas becomes more difficult and expensive using a cosolvent.

A pressurized gas with high solubility for most of the pharmaceutical compounds would allow the process to be effected without the use of cosolvents.

The object of the present invention is thus to provide a novel process for producing (sub)micron-sized particles of a biologically active compound selected from the group consisting of Saquinavir, Orlistat, Isotretinoin, Sulfamethoxazol, Diazepam, Moclobemide and Bosentan, (pharmaceutical) from a compressed dimethyl ether containing a surface modifier selected from the group consisting of polyethylenglycol ether of -lauryl, -cetyl, -stearyl and - oleylalcohols or sodium di-isooctyl-sulphosuccinate, avoiding the above mentioned difficulties.

WO-A-9521688 disclosed the preparation of particles from a supercritical gas. However, said particles are not submicronized particles. This document also fails to teach the use of dimethylether and of the surface modifier according to the invention.

US-A-4,734,227 pertains to the preparation of polymeric films or of submicron sized powders. US-A-4,734,227 however discloses neither the use of dimethylether, nor the use of the abovementioned specific surface modifiers, nor the use of an antisolvent.

US-A-3,998,753 relates to conventional spray-drying techniques without the recourse to supercritical or compressed fluids, which technique is well known but has almost nothing to do with spray-drying techniques with supercritical/compressed fluids. Further, to this document does not pertain to the preparation of submicronized particles.

Although Pharm. Acta. Helv., 66, Nr. 5-6, 1991, pages 170-173 makes reference to previous articles pertaining to the preparation of submicronized powders, the document itself does not relate to the preparation of submicronized powders, but to micronization using supercritical fluids. Moreover, it discloses neither the use of dimethylether, nor the use of the specific claimed surface modifiers, nor the use of an antisolvent.

As for WO-A-9816204 of which the Applicant is proprietor, it does not disclose the use of a surface modifier selected from:
- either a polyethylenglycol ether of -lauryl,-cetyl, -stearyl and - oleylalcohols, or
- a sodium di-isooctylsulphosuccinate.

The process of the present invention is based on the use of compressed dimethylether together with the above-mentioned surface modifier including supercritical technology yielding (sub)micron-sized particles having a narrow size distribution and being stabilized by a surface modifier.

The suggested process can be performed either batchwise or continuously and is applicable to a wide range of substances.

In a first aspect of the invention it has now been found that the above mentioned problems concerning the cosolvent can be avoided by using compressed dimethylether to solve the biologically active compound.

In a second aspect of the invention it has now been found that the above mentiond problems of clogging can be avoided by stabilizing the supercritical solution by adding (the above-mentioned) surface modifier in the compressed dimethylether solution.

The invention thus concerns a process for the manufacture of a pulverous preparation of a (sub)micron-sized biologically active compound selected from the group consisting of Saquinavir, Orlistat, Isotretinoin, Sulfamethoxazol, Diazepam, Moclobemide, and Bosentan comprising the steps of:
(1) dissolving said biologically active compound under elevated pressure in compressed dimethylether containing a surface modifier selected from the group consisting of a polyethylenglycol ether of -lauryl,-cetyl, -stearyl and - oleylalcohols or sodium di-isooctylsulphosuccinate;
(2a) rapidly expanding the compressed solution of step (1) thereby precipitating the dissolved compound; or
(2b) spraying the compressed solution of step (1) into an antisolvant selected from compressed carbon dioxide or water optionally containing a surface modifier selected from the group consisting of a polyethylenglycol ether of -lauryl,-cetyl, -stearyl and -oleylalcohols or sodium di-isooctylsulphosuccinate; under vacuum, atmospheric pressure or elevated pressure;
(3) optionally converting the antisolvant phase of step (2b) into a pulverous preparation using conventional powder processing techniques.

Convential powder techniques are for example spray drying and freeze drying.

In this manner the formation of (sub)micron sized particles stabilized by a surface modifier is achieved.

The term "(sub)micron-sized particles" embraces particles having a medium diameter (Dv 0.5) of 5nm to 5µm, preferably 200nm to 1 µm.

The surface modifiers used according to the invention are Brij 96® (polyethylenglykolether of lauryl,-cetyl-, stearyl- and oleylalcohols, available from Atlas Chemie) and Aerosol OT® (sodium di-isooctylsulphosuccinate availaible from Wako Junyaku Corp).

In step (1) and step (2b) of the process the same modifier can be used.

As shown by H. Steffen (BT Gattefossé No. 81, 1988, pp. 45-53,) the concentration of the surface modifier depends on the critical micelle concentration (CMC). The amount of surface modifier needed depends therefore on the CMC and the surface aerea of the particles.

Due to the presence of a surface modifier in the compressed dimethylether the following advantages are achieved:
- Differences of the pressure and temperature are counteracted by stabilizing any nuclei formed.
- The pressure drop in the region of the nozzle can be accommadated without clogging.
- The surface modifier is located very close to the region of particle formation and not distributed in the whole liquid.
- It is possible to expand into a liquid phase (e.g. compressed CO₂), which is then evaporated by keeping the stabilization of the suspension. Thus, the additional step of spray drying is no longer necessary.

The term "biologically active compound" includes e.g.pharmaceuticals as listed below:
- therapeutic category: INN (international non-proprietary name)
- anxiolytic: Diazepam, Bromazepam
- antidepressant: Moclobemide
- anesthetic: Midazolam
- antiviral: Ganciclovir, Zalcitabine, Nelfinavir mesylate
- proteinase inhibitor: Saquinavir, Nelfinavir
- anti-inflammatory: Naproxen, Tenoxicam, Ketorolac
- antibacterial: Ceftriaxone,
Timethoprim,
Sulfamethoxazol.
- antimalarial: Mefloquine
- antihypertensive: Cilazapril
- antiseborrheic: Isotretinoin
- calcium regulator: Calcitriol
- lipase inhibitor: Orlistat
- antiparkinson: Tolcapone
- antiarthritic: Mycophenolate mofetil
- antithrombotic: Lamifiban
- endothelin antagonist: Bosentan

### The antisolvant is water or compressed CO₂

The temperature in steps (1) or (2b) is each independently in the range of 0-250°C, preferably 20-60°C.

The pressure in step (1) is 2-500x10⁵ Pa, preferably 2-300x10⁵ Pa and the pressure in step (2b) is 0.05-500x10⁵ Pa, preferably 1-200x 10⁵ Pa, most preferably 3-100x10⁵ Pa.

Preferably the pressure in step (1) and step (2b) is not the same. The pressure difference is used to control the particle size.

The invention is further explained with reference to the attached drawings in which
- Fig. 1 is a schematic representation of an apparatus for carrying out the present invention;
- Fig 2 and Fig.3 show the particle size distribution of the same suspension but using different methods to determine the particle size distribution.

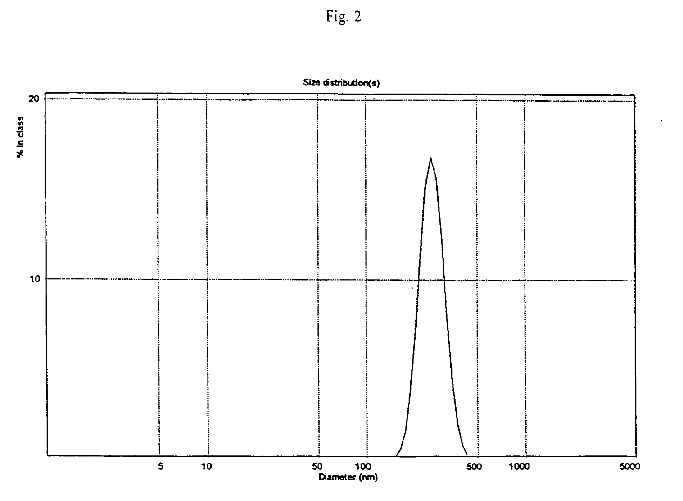

Fig. 2 shows the particle size distribution of Saquinavir using Photon Correlation Spectroscopy (batch no. 1051/30) ; Rec 7; Angle 90; KCps 931.3; Zave 254.7; Poly 0.031; Multi Angle.

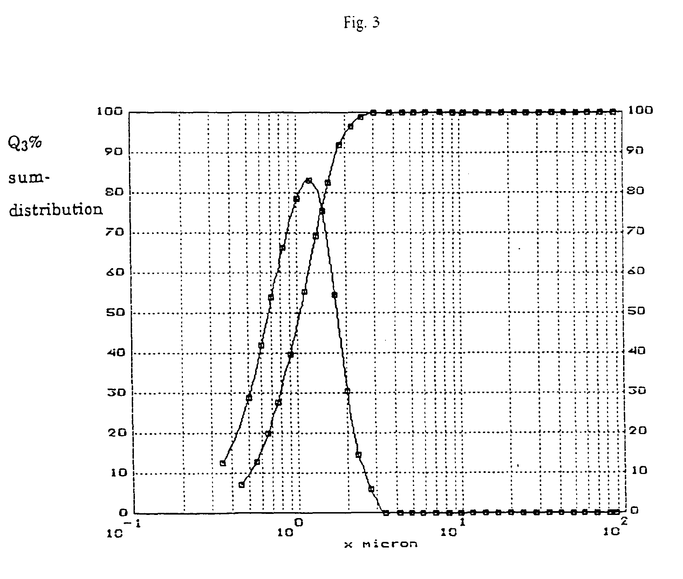

Fig. 3 shows particle size distribution of Saquinavir using Laser Diffraction (batch no. 1051/30); modifier Aerosol OT; dimethylether; focus 50mm;

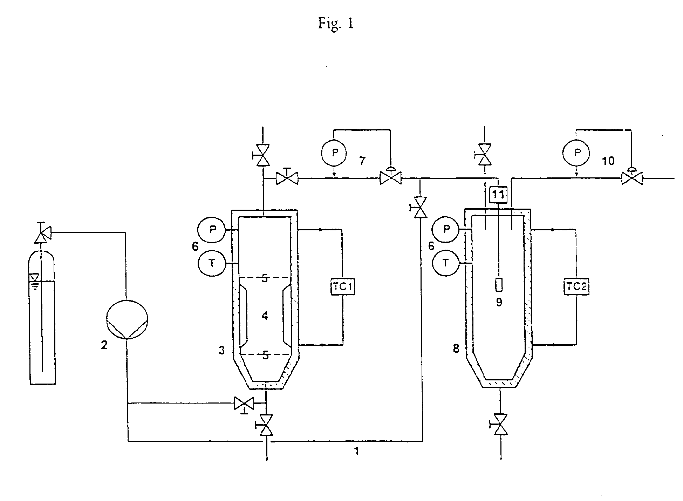

Fig. 1 is described as follows:

A 61 high pressure vessel (3) for dissolving the drug substance (and optionally the surface modifier) was connected via an outlet tube to a 41 high pressure vessel (8) which was used as the precipitation unit. The dissolution unit (3) was equipped with a container (4) closed with two sinter plates (5) which retained the solid drug substance (and if present the solid surface modifier) but allowed free flow of the compressed fluid and drug (and optionally surface modifier) / compress fluid solution through it. A bypass line (1) allowed to pre-pressurize the precipitation unit (8). The temperature of the two vessels (3) and (8) was controlled independently of each other by two thermostates TC1 and TC2. All pipes were heated by heating tape. The pressure in the two vessels (3, 8) was controlled using two pressure regulators (7, 10). The flow rate through the nozzle (9) was measured with a flow meter (11). The expansion nozzle included a 1.5 mm thick, 0.1mm diameter laser drilled orifice. The downstream end of the orifice was counterbored into a V-shape.

A typical experiment consisted of:
(i) charging the container (4) with the desired amount of drug substance and (optionally) surface modifier,
(ii) closing the container with the sinter plates (5) and putting it into vessel,
(iii) adding the antisolvent (optionally together with surface modifier) to the precipitation unit (8),
(iv) pressureizing the two vessels (3) and (8) on the desired pressure levels, and
(v) thermostating the vessels and the pipes on the desired temperature levels.

The whole system was equilibrated (e.g. 90 min), after which the spraying process was started by pumping additional compressed fluid into vessel (3). The increase of the pressure in the dissolution chamber (3) forced the pressure regulator (7) to open the valve to the spraying unit thereby starting the spraying. The differential pressure between the first (3) and second (8) vessel was controlled by a pressure regulator (10). The flow rate through the nozzle (9) was controlled by adjusting the pump flow rate (2). During the whole experiment, temperature and pressure in the two vessels (3, 8) were monitored constantly.

A continuous process can be achieved by continuous, controlled feeding of drug substance (and optionally surface modifier) into the dissolution unit (3), dissolving it in the compressed fluid and spraying the solution into the antisolvent phase in the precipitation unit (8). Suspension is continually removed from the precipiation unit and replaced by new antisolvent (optionally containing surface modifier).

The particle size distribution of very small particles of approximately 1 µm is very difficult to determine accurately. In principle there are two different methods commonly used, photon correlation spectroscopy (PCS) and laser diffraction. Photon correlation spectroscopy is commonly used for characterization of submicron suspensions and emulsions. Due to the principle of the measurement (movement of particles) particles larger than 3 to 5 µm cannot be seen with this method. With laser diffraction small particles (>0.1 µm) as well as larger particles (up to 2 mm) can be characterized in parallel. The diffraction of the light is thereby measured at small diffraction angles. For very small particles the method tends to overestimate the particle size due to transition of light through the particles. This effect of over- and underestimation of the particle size by the two methods is demonstrated in Figure 2 and Figure 3, showing the particle size distribution of the same suspension, measured with PCS (Figure 2), and with laser diffraction (Figure 3).

To assess the performance of a process for the formation of (sub)micron-sized particles, it is important to show that - besides the fine particles - no fractions of large particles are formed. Formation of fractions of coarse particles was observed especially after clogging of the nozzle (e.g. expansion of a compound dissolved in a compressed gas without modifier). To be able to detect the presence of coarse particles, laser diffraction was chosen to characterize the whole suspension. With its wide dynamic range, laser diffraction allowes the detection of particles up to 2 mm that cannot be seen by the PCS method. Since laser diffraction tends to overestimate the particle size (Figures 2, 3), all the particle sizes determined by laser diffraction can be considered as too large. Nevertheless laser diffraction proved to be sensitive enough to show the influence of different process parameters on the particle size.

The following Examples explain the invention in more detail, e.g. comparing the solubilities of pharmaceutical drug substances in dimethyl ether (according to the invention) and in carbon dioxide (not according to the invention). (example 1)

### Example 1: . Solubilities of pharmaceutical drug substances in liquid carbon dioxide and dimethyl ether

A comparison of solubilities of a number of pharmaceutical drug substances was performed as follows:

App. 3-5 g of the drug product was slightly compressed in an uniaxial press to avoid the formation of a stable suspension. The so compressed powder was given in a pressure chamber with a sapphire glass (30 ml volume). The temperature of the pressure chamber was controlled by water bath. Then the pressure in the chamber was increased using the corresponding gas and equilibrated for 1-3 hours. After equilibration a defined sample (1.0 ml) was drawn under constant pressure and temperature conditions using a high pressure line with a defined volume. This sample was expanded into a liquid with a good solubility for the respective compound. The sample container was afterwards rinsed with the same liquid to collect the residues of the substance in the sample container.

The solubility (G/V) was determined either by HPLC or gravimetrically after removing the liquid.

Solubilities of pharmaceutical drug substances in liquid carbon dioxide and dimethyl ether

| drug substance | solubility | conditions | solubility | conditions |
|---|---|---|---|---|
| | (CO₂) [%(g/V)] | [°C/bar] | (DME) [%(g/V)] | [°C/bar] |
| Orlistat (THL) | 0.6 | 30°C/100 bar | 17.8 | 20°C /4.5 bar |
| Isotretionin | 0.3 | 45°C/200 bar | 6.0 | 45/200 bar |
| Sulfamethoxaz ol | 0.1 | 45°C/140 bar | 5.4 | 45°C/140 bar |
| Saquinavir | < 0.1 | 45°/200 bar | >10 | 25°C/100 bar |
| Diazepam | 0.15 | 45°C/200 bar | > 10 | 45°C/200 bar |
| Moclobemide | 0.35 | 45°/200 bar | 3.7 | 45°C/200 bar |
| Bosentan | <0.1 | 45°C/200 bar | 9.0 | 45°C/200 bar |

### Example 2: Expansion of Orlistat (Tetrahydrolipstatin THL) - Influence of the spraying time (not according to the invention).

150 g of solid THL and 75 g Brij 96 in a container with two sinter plates was charged into an autoclave having a volume of 61. The autoclave was kept at a temperature of 40°C with a water bath. Then the autoclave was filled with CO₂ up to a pressure of 200 bar and equilibrated for 90 min.

The autoclave was connected to a second autoclave via a heated high pressure line, kept at 40°C. This second autoclave had a volume of 4 1. The dissolved THL was sprayed into a 1.251 of an aqueous solution (0.06% =1 CMC) this second autoclave. Thereby the pressure of the first autoclave was kept constant at 200 bar by pumping in additional gas.
(Several trials spraying a solution of THL in CO₂ without surfactant into an aqueous solution with various concentrations of Brij 96 was not successful due to clogging of the nozzle. The small amount of surfactant (1 CMC) was not added for stabilization.).

After 90,150 , and 180 min spraying a sample for particle size distribution was drawn. After 180 min the whole amount of THL/Brij was removed from the first container (≈ 12%THL in the final suspension). That means that a solid concentration of 5-8 % should be achievable in production scale.

As listed below the resulting particle size distribution of THL was kept almost constant over the whole trial (see table below). This shows that stabilization of the nuclei with the surfactant was very effective up to a high solid concentration. This fact is a prerequisite for an effective process.

Particle size distributions determined with laser diffraction.

| Spraying Time | Dv 0.1 [µm] | **Dv 0.5 [µm]** | Dv 0.9 [µm] |
|---|---|---|---|
| 90 min | 0.6 | **1.4** | 2.9 |
| 150 | 0.4 | **1.5** | 3.5 |
| 180 | 0.9 | **2.1** | 4.5 |

### Example 3: Expansion of Saquinavir - Influence of the pressure in the first container on the resulting particle size (according to the invention).

50 g of solid Saquinavir and 25 g Aerosol OT in a container with two sinter plates was charged into an autoclave having a volume of 6 1. The autoclave was kept at a temperature of 40°C with a water bath. Then the autoclave was filled with DME up to different pressures and equilibrated for 90 min.

The autoclave was connected to a second autoclave via a heated high pressure line, kept at 25°C, 5 bar. This second autoclave had a volume of 41. The dissolved Saquinavir/Aerosol OT was sprayed into a second autoclave filled with 1.21 of pure water. Thereby the pressure of the first autoclave was kept constant by pumping in additional gas.
(Several trials spraying a solution of Saquinavir in DME without surfactant into an aqueous solution with various concentrations of surfactant was not successful due to clogging of the nozzle)

After 20 min spraying a sample for particle size distribution was drawn. (≈ 4% Saquinavir in the final suspension).

The resulting particle size distribution of Saquinavir could be controlled by the pressure applied in the first container (see table below). This shows that as theoretically proposed the supersaturation can be kept very constant during the process and correlates with the resulting particle size. Also the stabilization of the nuclei with the surfactant was very effective. This fact is also a prerequisite for a effective and controlled process.

The nozzle diameter was 0.1 mm. As commonly known a further decrease of particle size can be obtained by decrease of the nozzle diameter.

Particle size distributions determined with laser diffraction.

| Pressure | Dv 0.1 [µm] | **Dv 0.5 [µm]** | Dv 0.9 [µm] |
|---|---|---|---|
| 50 bar | 0.5 | **3.8** | 6.4 |
| 100 bar | 1.0 | **2.1** | 4.5 |
| 200 bar | 0.9 | **1.5** | 2.4 |
| 280 bar | 0.4 | **0.8** | 1.7 |

### Example 4: Expansion of Saquinavir - Influence of the surfactant (according to the invention).

50 g of solid Saquinavir and 5 g Brij 96 in a container with two sinter plates was charged into an autoclave having a volume of 61. The autoclave was kept at a temperature of 40°C with a water bath. Then the autoclave was filled with DME up to 200 bar and equilibrated for 90 min.

The autoclave was connected to a second autoclave via a heated high pressure line, kept at 25°C, at 5 bar. This second autoclave had a volume of 4 1. The dissolved Saquinavir/Brij 96 was sprayed into a second autoclave filled with 1.21 of pure water. Thereby the pressure of the first autoclave was kept constant by pumping in additional gas.

After 20 min spraying a sample for particle size distribution was drawn. (≈ 4% Saquinavir in the final suspension).

The resulting particle size distribution of Saquinavir stabilized with Brij 96 (not ionic surfactant) was comparable with the results obtained with Aerosol OT (ionic surfactant, see Example 3).
Particle size distributions determined with laser diffraction .

| Surfactant | Dv 0.1 [µm] | **Dv0.5[µm]** | Dv 0.9 [µm] |
|---|---|---|---|
| Aerosol OT | 0.9 | **1.5** | 2.4 |
| Brij 96 | 0.7 | **1.4** | 3.0 |

## Claims

1. A process for the manufacture of a pulverous preparation of a (sub)micron-sized biologically active compound selected from the group consisting of Saquinavir, Orlistat, Isotretinoin, Sulfamethoxazol, Diazepam, Moclobemide, and Bosentan comprising the steps of:
(1) dissolving said biologically active compound under elevated pressure in compressed dimethylether containing a surface modifier selected from the group consisting of a polyethylenglycol ether of -lauryl,-cetyl, -stearyl and -oleylalcohols or sodium di-isooctylsulphosuccinate;
(2a) rapidly expanding the compressed solution of step (1) thereby precipitating the dissolved compound; or
(2b) spraying the compressed solution of step (1) into an antisolvant selected from compressed carbon dioxide or water optionally containing a surface modifier selected from the group consisting of a polyethylenglycol ether of -lauryl,-cetyl, -stearyl and - oleylalcohols or sodium di-isooctylsulphosuccinate; under vacuum, atmospheric pressure or elevated pressure;
(3) optionally converting the antisolvant phase of step (2b) into a pulverous preparation using conventional powder processing techniques.

2. A process according to claim 1, wherein the temperature in step (1) or (2b) is each independently in the range of 0-250°C, preferably 20-60 °C.

3. A process according to any one of claim 1 or 2, wherein the pressure in step (1) is 2-500x10⁵ Pa, preferably 2-300x10⁵ Pa and the pressure in step (2b) is 0.05-500x10⁵ Pa, preferably 1-200x10⁵ Pa, most preferably 3-100x10⁵ Pa.

4. A process according to claim 3, wherein a pressure difference exists between step (1) and step (2), said pressure difference being used to control the particle size.

5. A process according to any one of claims 1-4, wherein the particles have a medium diameter (Dv 0.5) of 5nm to 5µm, preferably 200nm to 1µm.

6. A process according to any one of claims 1-5, wherein the antisolvant phase is compressed CO₂ and the pulverous preparation is obtained by evaporating the antisolvant phase to atmospheric pressure.

7. A process according to any one of claims 1-6, wherein the process is performed batchwise.

8. A process according to any one of claims 1-7, wherein the process is performed continuously comprising the steps of:
(1) controlled feeding of a biologically active compound selected from the group consisting of Saquinavir, Orlistat, Isotretinoin, Sulfamethoxazol, Diazepam, Moclobemide, and Bosentan and a surface modifier selected from the group consisting of a polyethylenglycol ether of -lauryl,-cetyl, -stearyl and -oleylalcohols or sodium di-isooctylsulphosuccinate into a dissolution unit;
(2) dissolving said biologically active compound in a compressed dimethylether and spraying the solution into an antisolvent selected from compressed carbon dioxide or water in a precipitation unit;
(3) continually removing the suspension from the precipitation unit and replacing the suspension by new antisolvent selected from compressed carbon dioxide or water optionally containing a surface modifier selected from the group consisting of a polyethylenglycol ether of -lauryl,-cetyl, -stearyl and -oleylalcohols or sodium di-isooctylsulphosuccinate.

## Patentansprüche

1. Verfahren zur Herstellung eines pulverförmigen Präparates einer (sub)mikrometergroßen biologisch aktiven Verbindung, ausgewählt aus der Gruppe, bestehend aus Saquinavir, Orlistat, Isotretinoin, Sulfamethoxazol, Diazepam, Moclobemid und Bosentan, umfassend die Schritte:
(1) Lösen der biologisch aktiven Verbindung unter erhöhtem Druck in komprimiertem Dimethylether, enthaltend einen Oberflächenmodifikator, ausgewählt aus der Gruppe, bestehend aus einem Polyethylenglykolether von Lauryl-, Cetyl-, Stearyl- und Oleylalkoholen oder Natriumdiisooctylsulfosuccinat;
(2a) schnelles Expandieren der komprimierten Lösung aus Schritt (1), wodurch die gelöste Verbindung ausfällt; oder
(2b) Sprühen der komprimierten Lösung aus Schritt (1) in ein Antisolvent, ausgewählt aus komprimiertem Kohlendioxid oder Wasser, gegebenenfalls enthaltend einen Oberflächenmodifikator, ausgewählt aus der Gruppe, bestehend aus einem Polyethylenglykolether von Lauryl-, Cetyl-, Stearyl- und Oleylalkoholen oder Natriumdiisooctylsulfosuccinat; unter Vakuum, Atmosphärendruck oder erhöhtem Druck;
(3) gegebenenfalls Umwandeln der Antisolventphase aus Schritt (2b) in ein pulverförmiges Präparat unter Verwendung konventioneller Pulververarbeitungstechniken.

2. Verfahren nach Anspruch 1, worin die Temperatur in Schritt (1) oder (2b) jeweils unabhängig voneinander im Bereich von 0 bis 250 °C, vorzugsweise 20 bis 60 °C, liegt.

3. Verfahren nach einem der Ansprüche 1 oder 2, worin der Druck in Schritt (1) 2 bis 500 x 10⁵ Pa, vorzugsweise 2 bis 300 x 10⁵ Pa, beträgt und der Druck in Schritt (2b) 0,05 bis 500 x 10⁵ Pa, vorzugsweise 1 bis 200 x 10⁵ Pa, am stärksten bevorzugt 3 bis 100 x 10⁵ Pa, beträgt.

4. Verfahren nach Anspruch 3, worin zwischen Schritt (1) und Schritt (2) ein Druckunterschied vorliegt, wobei dieser Druckunterschied zur Regelung der Teilchengröße verwendet wird.

5. Verfahren nach einem der Ansprüche 1 bis 4, worin die Teilchen einen mittleren Durchmesser (Dv 0,5) von 5 nm bis 5 µm, vorzugsweise 200 nm bis 1 µm, haben.

6. Verfahren nach einem der Ansprüche 1 bis 5, worin die Antisolventphase komprimiertes CO₂ ist und das pulverförmige Präparat erhalten wird, indem die Antisolventphase bei Atmosphärendruck eingedampft wird.

7. Verfahren nach einem der Ansprüche 1 bis 6, worin das Verfahren diskontinuierlich durchgeführt wird.

8. Verfahren nach einem der Ansprüche 1 bis 7, worin das Verfahren kontinuierlich durchgeführt wird, umfassend die Schritte:
(1) kontrolliertes Zuführen einer biologisch aktiven Verbindung, ausgewählt aus der Gruppe, bestehend aus Saquinavir, Orlistat, Isotretinoin, Sulfamethoxazol, Diazepam, Moclobemid, und Bosentan, und eines Oberflächenmodifikators, ausgewählt aus der Gruppe, bestehend aus einem Polyethylenglykolether von Lauryl-, Cetyl-, Stearyl- und Oleylalkoholen oder Natriumdiisooctylsulfosuccinat, zu einer Auflösungseinheit;
(2) Lösen der biologisch aktiven Verbindung in einem komprimierten Dimethylether und Sprühen der Lösung in ein Antisolvent, ausgewählt aus komprimiertem Kohlendioxid oder Wasser in einer Ausfällungseinheit;
(3) kontinuierliches Entfernen der Suspension aus der Ausfällungseinheit und Ersetzen der Suspension durch neues Antisolvent, ausgewählt aus komprimiertem Kohlendioxid oder Wasser, gegebenenfalls enthaltend einen Oberflächenmodifikator, ausgewählt aus der Gruppe, bestehend aus einem Polyethylenglykolether von Lauryl-, Cetyl-, Stearyl- und Oleylalkoholen oder Natriumdiisooctylsulfosuccinat.

## Revendications

1. Procédé de fabrication d'une préparation pulvérulente d'un composé biologiquement actif de taille (sub)micronique choisi dans le groupe constitué par le saquinavir, l'orlistat, l'isotrétinoïne, le sulfaméthoxazole, le diazépam, le moclobémide et le bosentan, comprenant les étapes consistant à :
(1) dissoudre ledit composé biologiquement actif sous pression élevée dans du diméthyléther comprimé contenant un modificateur de surface choisi dans le groupe constitué par un éther de polyéthylène glycol avec l'alcool laurylique, cétylique, stéarylique et oléylique ou par le diisooctylsulfosuccinate de sodium;
(2a) expandre rapidement la solution comprimée de l'étape (1) afin de précipiter le composé dissous; ou
(2b) pulvériser la solution comprimée de l'étape (1) dans un antisolvant choisi parmi du dioxyde de carbone comprimé ou de l'eau contenant le cas échéant un modificateur de surface, choisi dans le groupe constitué par un éther de polyéthylène glycol avec l'alcool laurylique, cétylique, stéarylique et oléylique ou par le diisooctylsulfosuccinate de sodium, sous vide, à la pression atmosphérique ou sous pression élevée ;
(3) convertir le cas échéant la phase antisolvant de l'étape (2b) en une préparation pulvérulente en utilisant des techniques classiques d'élaboration de poudres.

2. Procédé selon la revendication 1, dans lequel la température à l'étape (1) ou (2b) se situe indépendamment pour chacune dans la plage de 0 à 250°C, de préférence de 20 à 60°C.

3. Procédé selon l'une quelconque des revendications 1 ou 2, dans lequel la pression à l'étape (1) se situe dans la plage de 2 à 500 x 10⁵ Pa, de préférence de 2 à 300 x 10⁵ Pa, et la pression à l'étape (2b) se situe dans la plage de 0,05 à 500 x 10⁵ Pa, de préférence de 1 à 200 x 10⁵ Pa, de la façon la plus préférentielle de 3 à 100 x 10⁵ Pa.

4. Procédé selon la revendication 3, dans lequel il y a une différence de pression entre l'étape (1) et l'étape (2), ladite différence de pression étant utilisée pour contrôler la taille de particule.

5. Procédé selon l'une quelconque des revendications 1 à 4, dans lequel les particules ont un diamètre moyen (Dv 0,5) dans la plage de 5 nm à 5 µm, de préférence de 200 nm à 1 µm.

6. Procédé selon l'une quelconque des revendications 1 à 5, dans lequel la phase antisolvant est du CO₂ comprimé et la préparation pulvérulente est obtenue en évaporant la phase antisolvant à la pression atmosphérique.

7. Procédé selon l'une quelconque des revendications 1 à 6, dans lequel le procédé se fait par lots.

8. Procédé selon l'une quelconque des revendications 1 à 7, lequel procédé s'effectuant en continu, comprenant les étapes consistant à :
(1) alimenter de manière contrôlée un composé biologiquement actif, choisi dans le groupe constitué par le saquinavir, l'orlistat, l'isotrétinoïne, le sulfaméthoxazole, le diazépam, le moclobémide et le bosentan et un modificateur de surface, choisi dans le groupe constitué par un éther de polyéthylène glycol avec l'alcool laurylique, cétylique, stéarylique et oléylique ou par le diisooctylsulfosuccinate de sodium, dans un appareil de dissolution ;
(2) dissoudre ledit composé biologiquement actif dans du diméthyléther comprimé et pulvériser la solution dans un antisolvant, choisi parmi du dioxyde de carbone comprimé ou de l'eau, dans un appareil de précipitation ;
(3) enlever en continu la suspension de l'appareil de précipitation et remplacer la suspension par un nouvel antisolvant choisi parmi du dioxyde de carbone comprimé ou de l'eau et contenant le cas échéant un modificateur de surface choisi dans le groupe constitué par un éther de polyéthylène glycol avec l'alcool laurylique, cétylique, stéarylique et oléylique ou par le diisooctylsulfosuccinate de sodium.
